(19) **Europäisches Patentamt / European Patent Office / Office européen des brevets**

(11) **EP 3 264 078 B1**

(12) **EUROPEAN PATENT SPECIFICATION**

(45) Date of publication and mention of the grant of the patent:
**10.11.2021 Bulletin 2021/45**

(21) Application number: **16290120.1**

(22) Date of filing: **29.06.2016**

(51) Int Cl.:
*G01N 33/28* *(2006.01)*  *E21B 43/34* *(2006.01)*

(54) **DETERMINING EMULSION PROPERTIES USING ELECTROMAGNETIC WAVES**

BESTIMMUNG VON EMULSIONSEIGENSCHAFTEN MITTELS ELEKTROMAGNETISCHER WELLEN

DÉTERMINATION DES PROPRIÉTÉS D'UNE ÉMULSION UTILISANT DES ONDES ÉLECTROMAGNÉTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**03.01.2018 Bulletin 2018/01**

(73) Proprietors:
• **Services Pétroliers Schlumberger**
**75007 Paris (FR)**
Designated Contracting States:
**FR**
• **SCHLUMBERGER TECHNOLOGY B.V.**
**2514 JG The Hague (NL)**
Designated Contracting States:
**AL AT BE BG CH CY CZ DE DK EE ES FI GB GR HR HU IE IS IT LI LT LU LV MC MK MT NL NO PL PT RO RS SE SI SK SM TR**

(72) Inventors:
• **ALLOUCHE, Francis**
**92140 Clamart (FR)**

• **JAN, Mathilde**
**92140 Clamart (FR)**

(74) Representative: **Schlumberger Intellectual Property Department**
**Parkstraat 83**
**2514 JG Den Haag (NL)**

(56) References cited:
**EP-A1- 1 593 418**   **US-A1- 2014 085 133**

• **T. KREBS ET AL: "Separation kinetics of an oil-in-water emulsion under enhanced gravity", CHEMICAL ENGINEERING SCIENCE, vol. 71, 1 March 2012 (2012-03-01), pages 118-125, XP055327226, GB ISSN: 0009-2509, DOI: 10.1016/j.ces.2011.10.057**
• **NILS-OLAV SKEIE ET AL: "Level estimation in oil/water separators based on multiple pressure sensors and multivariate calibration", JOURNAL OF CHEMOMETRICS, vol. 24, no. 7-8, 1 February 2010 (2010-02-01), pages 387-398, XP055160587, ISSN: 0886-9383, DOI: 10.1002/cem.1282**

**Description**

## BACKGROUND

**Field**

The present invention relates to the field of fluid property determination, and, more particularly, to the determination of properties of an emulsion phase of a multi-phase fluid, and the use of those determined properties for well testing and production well site design and operation.

**Description of the Related Art**

In oil production, transport, processing, and storage, multi-phase fluids are present. For example, a well may produce multiphase fluid having, for example, a gas phase, a water phase, and an oil phase. Some multiphase fluids may also have a solid phase including sand, mud, or other solid components. Although oil and water are immiscible, at the interface between the water phase and the oil phase an emulsion may form. That is, there may be a phase comprised of a mixture of water droplets in oil, water and oil droplets, and oil droplets and water. Other interface phases may also be present in a multiphase fluid, such as between the oil and gas phase or even between a solid phase and any of the multiphase fluids.

The determination of properties of the emulsion or any of the interface phases may be useful for a variety of reasons relating to storage, transport, or separation of the phases. Current techniques and technologies, however, may not provide for accurate determination of such properties. To that end, further development, both of technologies to accurately determine these properties, as well as new ways of using such accurately determined properties, is desirable.

US 2014/0085133 describes a radar transmitter for emulsion measurement comprising a probe defining a transmission line for sensing impedance. A pulse circuit is connected to the probe for generating pulses on the transmission line and receiving a reflected signal from the transmission line. The reflected signal comprises a waveform of probe impedance over time. A controller, operatively connected to the pulse circuit profiles content of the emulsion responsive to the waveform by transforming the waveform into impedance relative to distance, converting the transformed waveform into effective dielectric relative to distance, determining mixture content of the emulsion at select distances responsive to the effective dielectric at the select distances and developing an output representing mixture content relative to level units.

## SUMMARY

Certain aspects of some embodiments disclosed herein are set forth below. It should be understood that these aspects are presented merely to provide the reader with a brief summary of certain forms the invention might take and that these aspects are not intended to limit the scope of the invention. Indeed, the invention may encompass a variety of aspects that may not be set forth below.

The present invention resides in a method of determining properties of a fluid having an oil phase, a water phase, and an emulsion phase between the oil phase and water phase and in an apparatus including a vessel containing such a fluid as defined in the appended claims.

Various refinements of the features noted above may exist in relation to various aspects of the present embodiments. Further features may also be incorporated in these various aspects as well. These refinements and additional features may exist individually or in any combination. For instance, various features discussed below in relation to the illustrated embodiments may be incorporated into any of the above-described aspects of the present disclosure alone or in any combination. Again, the brief summary presented above is intended just to familiarize the reader with certain aspects and contexts of some embodiments without limitation to the claimed subject matter.

## BRIEF DESCRIPTION OF THE DRAWINGS

These and other features, aspects, and advantages of certain embodiments will become better understood when the following detailed description is read with reference to the accompanying drawings in which like characters represent like parts throughout the drawings.

FIG. 1 is a schematic block diagram of a fluid containing vessel and a radar associated therewith, in accordance with this disclosure.

FIG. 2 is a flowchart of a method of determining properties of an emulsion phase of a fluid, such as may be performed with the equipment of FIG. 1, in accordance with this disclosure.

FIG. 3 is a graph showing amplitude of reflected electromagnetic waves vs. which phase of a multi-phase liquid the reflections are from.

FIG. 4 is an example graph showing the relation between the water liquid ratio and the emulsion phase dielectric constant, for a sample multi-phase liquid.

FIG. 5 is a graph showing changes in position of the coalescing portion and the creaming portion of the emulsion phase of a multi-phase liquid over time.

FIG. 6 is a graph showing the percentage of emulsion separation of a multi-phase liquid over time.

FIG. 7 is a schematic block diagram of a wellsite production system employing the vessel and radar disclosed herein.

FIG. 8 is a flowchart of a method of using determined properties of an emulsion phase of the fluid.

FIG. 9 is a flowchart of another method of using determined properties of an emulsion phase of the fluid.

## DETAILED DESCRIPTION

The present description is made with reference to the accompanying drawings, in which example embodiments are shown. However, many different embodiments may be used, and thus the description should not be construed as limited to the embodiments set forth herein. Rather, these embodiments are provided so that this disclosure will be thorough and complete.

When introducing elements of various embodiments, the articles "a," "an," "the," and "said" are intended to mean that there are one or more of the elements. The terms "comprising," "including," and "having" are intended to be inclusive and mean that there may be additional elements other than the listed elements. Moreover, any use of "top," "bottom," "above," "below," other directional terms, and variations of these terms is made for convenience, but does not mandate any particular orientation of the components.

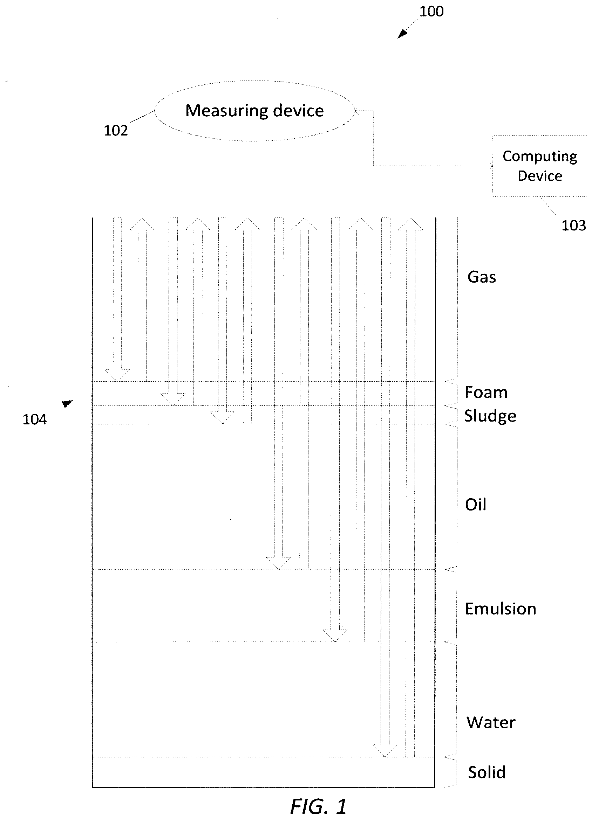

With reference to FIG. 1, a system 100 for determining properties of a fluid is now described. The fluid is contained within a vessel or body 104, which may be a vessel or body of any sort and take any shape. For example, the vessel or body 104 may be a closed tank, closed pipe, production separator, or testing separator.

The fluid has a solid phase, a water phase, an oil, phase, an emulsion phase between the water phase and oil phase, a sludge phase, a foam phase, and a gas phase. It should be understood that some of these phases may not be present, and that Docket No.: IS15.0204-EP-EPA any combination of these phases may be present. The emulsion phase is heterogeneous, having varying amount of water and oil droplets at different locations.

A measuring device 102 is aimed at the vessel 104, and a computing device 103 is coupled to the measuring device 102 for control thereof and data collection therefrom. The measuring device 102 is a guided wave radar. The measuring device 102 may even include multiple of these types of devices.

The computing device 103 may be a microprocessor, microcontroller, system on a chip, programmed logic controller, field programmable gate array, or other suitable programmable device. It should be appreciated that the computing device 103 has been programmed to perform the functions taught herein, and that such programming causes a change in the physical characteristics of the computing device 103 over a generic unprogrammed computing device. For example, in the case where the computing device 103 is a microprocessor constructed from transistors, the programming changes the biasing of the transistors, which causes physical changes in the semiconductor material of the transistors. In the case where the computing device 103 is a programmed logic controller or field programmable gate array, the programming changes the configuration of the logic gates in the computing device 103.

Example operation will be explained below where the measuring device 102 is a guided wave radar, although as stated above, in some configurations it may not be a guided wave radar. The computing device 103, as will be described below, operates the radar 102 so as to emit electromagnetic waves into the fluid in the vessel 104. The different phases of the fluid each have a different dielectric constant, and thus the amplitude of reflections of the electromagnetic waves off each interface between different phases are different. In addition, by measuring the time elapsed from emission of an electromagnetic wave and receipt of a reflection thereof, the depth or thickness of a phase can be determined.

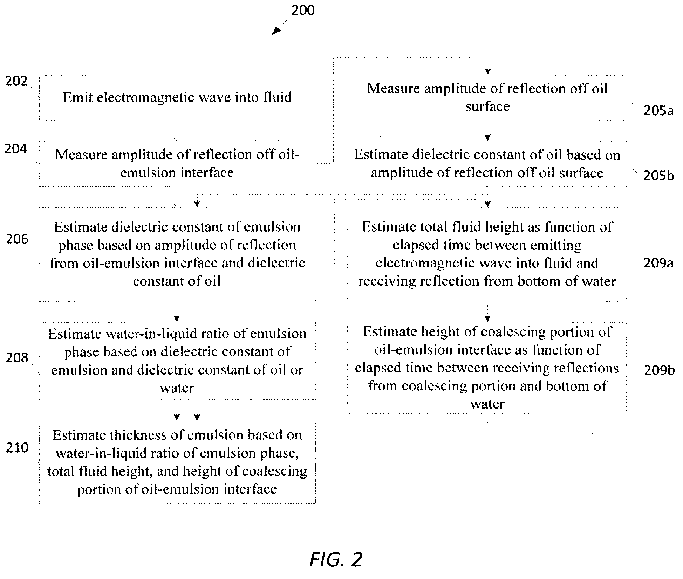

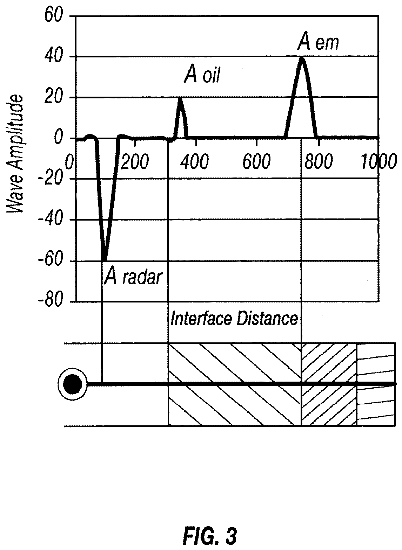

Operation of computing device 103 and radar 102 in order to determine properties of the emulsion phase will now be described with reference to the flowchart 200 of FIG. 2. The technique begins with the computing device 103 operating the radar 102 emit electromagnetic waves into the fluid vessel 104 (Block 202). Then, the computing device 103 measures the amplitude of the reflection of the electromagnetic waves off the interface between the oil phase and the emulsion phase (Block 204). Optionally, if the dielectric constant of the oil is not known, the computing device 103 measures the amplitude of the reflection of the electromagnetic waves off the surface of the oil, which can also be referred to as the interface between the oil and free space (Block .205a). Shown in FIG. 3 is a graph of the amplitude of the reflection from the surface of the oil and from the oil-emulsion interface, which illustrates the difference in amplitudes due to the differences in dielectric constant.

To estimate or determine the dielectric constant of the oil if not already known, the computing device 103, uses the amplitude of the reflection from the surface of the oil to together with the amplitude of the electromagnetic wave as emitted from the radar 102 (Block 205b). Mathematically, the amplitude of the oil is related to the dielectric constant of the oil as follows:

$$A_{oil} = \frac{1 - \sqrt{\varepsilon_{oil}}}{1 + \sqrt{\varepsilon_{oil}}} * A_{radar}$$

where $A_{oil}$ is the amplitude of the reflection from the surface of the oil, $A_{radar}$ is the amplitude of the electromagnetic wave as emitted from the radar, and $\varepsilon_{oil}$ is the dielectric constant of the oil.

Solving for the dielectric constant of the oil thus yields:

$$\varepsilon_{oil} = \frac{\left(\dfrac{A_{oil}}{A_{radar}} + 1\right)^2}{\left(\dfrac{A_{oil}}{A_{radar}} - 1\right)^2}$$

The computing device 103 then estimates or determines the dielectric constant of the emulsion phase from the amplitude of the reflection from the oil-emulsion interface and the electric constant of the oil (Block 206). Mathematically, the amplitude of the reflection from the oil-emulsion interface and dielectric constant of the oil are related to the dielectric constant of the emulsion phase as follows:

$$A_{em} = \frac{2}{1 + \sqrt{\varepsilon_{oil}}} \frac{\sqrt{\varepsilon_{oil}} - \sqrt{\varepsilon_{em}}}{\sqrt{\varepsilon_{oil}} + \sqrt{\varepsilon_{em}}} \frac{2\sqrt{\varepsilon_{oil}}}{1 + \sqrt{\varepsilon_{oil}}} * A_{radar}$$

where $A_{em}$ is the amplitude of the reflection from the oil-emulsion interface, $A_{radar}$ is the amplitude of the electromagnetic wave as emitted from the radar, $\varepsilon_{oil}$ is the dielectric constant of the oil, radar $\varepsilon_{em}$ is the dielectric constant of the emulsion.

Solving for the dielectric constant of the emulsion thus yields:

$$\varepsilon_{em} = \varepsilon_{oil} \frac{(\alpha - \beta)^2}{(\alpha + \beta)}, where\ \beta = \frac{A_{em}}{A_{radar}}, and\ where\ \alpha = \frac{4\sqrt{\varepsilon_{oil}}}{\left(1 + \sqrt{\varepsilon_{oil}}\right)^2}$$

Next, the computing device 103 determines or estimates the water-in-liquid ratio of the emulsion phase based on the dielectric constant of the emulsion and either the dielectric constant of the oil or the dielectric constant of water (Block 208). This correlation may be based on a model. Mathematically, if the dielectric constant of the emulsion and the dielectric constant of oil are used, the relationship is:

$$\varepsilon_{em} = \frac{\varepsilon_{oil}(1 + 2\emptyset_d)}{1 - \emptyset_d}, where\ \emptyset_d\ is\ the\ water - in - liquid\ ratio$$

Solving for the water-in-liquid ratio yields:

$$\emptyset_d = \frac{\varepsilon_m - \varepsilon_{oil}}{\varepsilon_m + 2\varepsilon_{oil}}$$

If the dielectric constant of the emulsion and the dielectric constant of water are used, the relationship is:

$$\varepsilon_{em} = \frac{\varepsilon_{water}(2\theta_d)}{3 - \theta_d}$$

Solving for the water-in-liquid ratio yields:

$$\emptyset_d = \frac{3\varepsilon_{em}}{\varepsilon_{em} + 2\varepsilon_{water}}$$

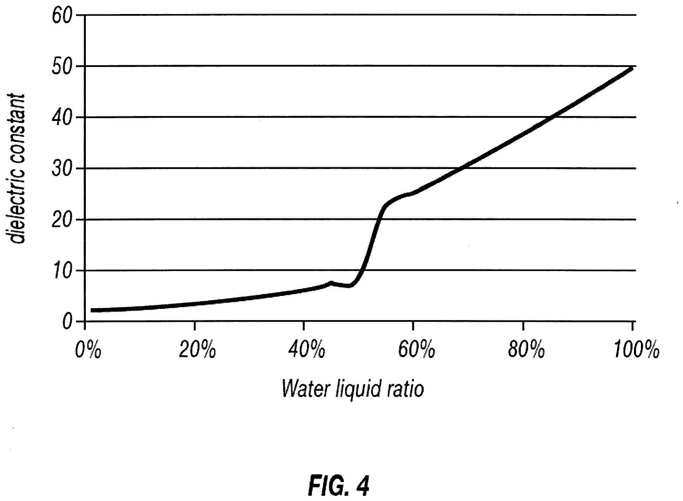

The relationship between the dielectric constant of an emulsion and a water-in-liquid ratio is shown, for a sample fluid, in FIG. 4. It should be appreciated that the relationship between the dielectric constant of the emulsion and the water-in-liquid ratio, as shown in FIG. 4, may be different for different fluids, depending on the dielectric constant of the oil, the dielectric constant of the water, and the water-in-liquid ratio at which the emulsion is changing from an oil-in-water emulsion to a water-in-oil emulsion.

Optionally, if not yet known, the total fluid height is estimated or determined by the computing device 103 (Block 209a). This is done by measuring the elapsed time between emission of the electromagnetic waves from the radar 102 and receiving a reflection of the electromagnetic waves from the bottom of the water (such as a reflection off the bottom of the vessel 104).

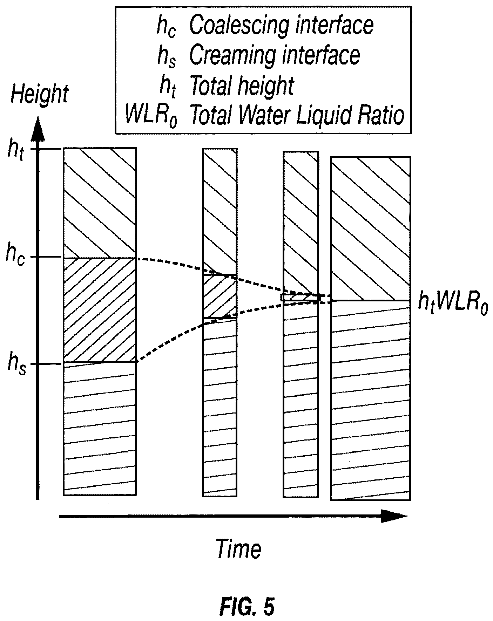

Those of skill in the art will understand that, just as the interface between the oil and water phases is not precise, resulting in the emulsion, the emulsion phase itself is not homogenous but is rather heterogeneous. The upper portion or interface of the emulsion phase where it meets the oil phase is called the coalescing interface, while the lower portion or interface of the emulsion phase where it meets the water phase is called the creaming interface. Over time the height between the coalescing interface and the creaming interface grows smaller, as shown in FIG. 5.

If not already known, the height of the coalescing portion is estimated or determined by the computing device 103 (Block 209b). This is done by measuring the elapsed time between emission of the electromagnetic waves and receiving the reflections from the coalescing portion.

Next, the computing device 103 estimates or determines the thickness of the emulsion phase based on the water-in-liquid ratio of the emulsion phase, the total fluid height, and the height of the coalescing portion of the oil-emulsion interface (Block 210). This estimation may be performed using a mass balance model. Mathematically, first

$$h_s = \frac{WLR_0 * h_t - WLR_{em} * h_c}{1 - WLR_{em}}$$

the height of the creaming interface is calculated as: , where $h_s$ is the height of the creaming interface, $h_c$ is the height of the coalescing interface, $h_t$ is the total fluid height, and $WLR_0$ is the total water liquid ratio.

The thickness of the emulsion phase is then calculated as:

$$Thickness_{em} = h_{\dot{c}} - h_s$$

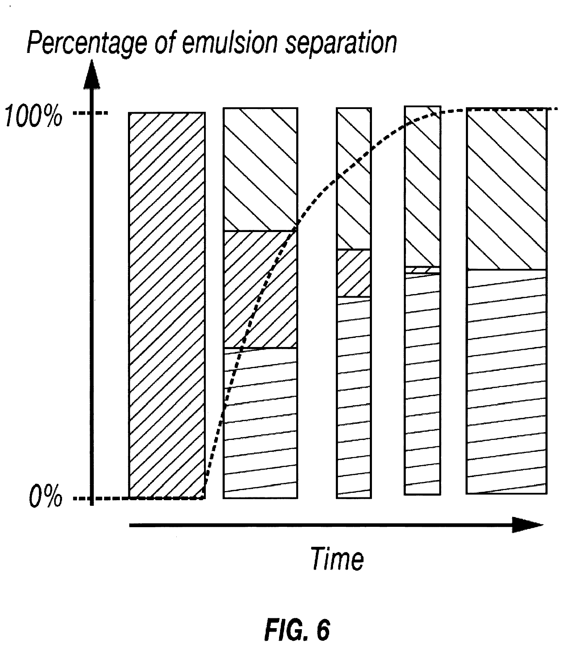

The thickness of the emulsion phase is useful in a variety of applications. As shown in FIG. 5, the thickness of the emulsion phase (difference between coalescing interface and creaming interface) subsides over time. This change in emulsion height or thickness over time can be interpreted as a percentage of emulsion separation or subsiding over time (i.e. rate of decrease in thickness over time), as shown in the graph of FIG. 6, allowing for estimation of the percentage of emulsion separation or subsiding over time. These estimations can be referred to as kinetics of separation.

Although the determination of the above characteristics and parameters has been described with reference to the emulsion phase, it should be understood that the techniques described above may be applied in the same way to the determination of those characteristics and parameters for any phase, such as the sludge phase.

In addition, although the determination of these various characteristics and parameters has been described above with respect to the use of a guided wave radar for measuring reflections, it should be appreciated that other techniques may be used to emit a signal into the fluid and measure properties from the various phases and the interfaces between them (including solids or solid phases). The properties of phases of a multiphase fluid (and any solids associated therewith) and interfaces between phases may be measured and determined by using reflection, refraction, and/or absorption techniques, or combinations thereof. The reflection, refraction and/or absorption of signals transmitted to and/or through the phases and interfaces may be measured by using signals all along the electromagnetic energy spectrum to determine the interface and phase properties of the multiphase fluid. The reflection, refraction and/or absorption of signals transmitted to and/or through the phases and interfaces may also be measured by using mechanical signals such as mechanically induced waves, for example sonar, acoustic waves, etc. The phase and interface properties may also be measured and determined by measuring electromagnetic properties of the phases and interfaces therebetween, such as, for example, inductance, conductance, capacitance, resistivity, etc.

Where the measured properties are determined from reflections from various phases and interfaces, similar calculations to those above can be applied to data from any such other technique. Where the measured properties are determined, for example, by signal refraction though the interfaces and/or the various phases, or by signal absorption through the interfaces and/or the various phases, other calculations and techniques may be used to determine the various characteristics and parameters of the fluids discussed above. Similarly, where the measured properties are determined using gamma ray emission or absorption by the interfaces and/or the various phases, neutron emission or absorption by the interfaces and/or the various phases, resistances of the interfaces and/or the various phases, relaxation times of hydrogen atoms of the interfaces and/or the various phases, or forces on the interfaces and/or the various phases due to gravity, other suitable calculations and techniques may be used to determine the various characteristics and parameters of the fluids discussed above. As explained above, some of the other measurement techniques may include radar, sonar, acoustic, resistivity, gravity, optical, nuclear magnetic resonance, and nuclear measurements.

Knowing the thickness of the interface phase, the settling time for the interface phase to subside, the water-in-liquid ratio of the interface phase, a height of a creaming interface of the interface phase, a height of a coalescing interface of the interface phase, and the kinetics of separation, each of which the techniques described above now make possible, is useful in a variety of applications. By kinetics of separation, times for, or rates at which, the interfaces decrease in thickness or subside is meant.

At the early stages of the process of exploring and appraising potential hydrocarbon bearing formations, a variety of information is gathered to verify the economic potential as well as the potential productivity of wells drilled in those potential hydrocarbon bearing formations. In gathering this information, formation fluids are quantitatively and qualitatively characterized, which in turn provides for the basis of the design of production operations to retrieve the hydrocarbons from the formation. The design includes the layout of wells themselves, and the architecture and layout of the surface production system with its interconnecting network of pipelines, production facilities, and export lines.

One major function of the production facilities is to separate the various phases of produced fluids. In order to optimize this separation, a proper and suitable size for the separators utilized over the total estimated production for the life of the formation is to be determined. Criteria utilized in sizing the separating devices is dependent on the type of separation processes being used (the main mechanisms that may be utilized include gravity, electrostatic, electromagnetic, thermal, centrifugal, and distillation).

Each type of separating process uses models to describe the kinetics of the separation between the phases. For example, Stoke's Law may be used to determine what retention times would be effective to achieve a desired degree of separation between a mixture of fluids of different densities, where gravity based separators are used. A useful parameter in this particular model is the size of the droplets of the discontinuous phase (such as the emulsion phase or sludge phase) that are to eventually float on or sink into the continuous phase (such as the water phase or the oil phase). Droplet size, however; is difficult to determine or estimate using conventional techniques.

Other parameters, such as the interfacial tension between the fluids also play a role in the separation model for the kinetics of gravity separators. Indeed, common sizing software for separator devices accepts as input a number of parameters that are either challenging to measure or unknown. Consequently, "rules of thumb," instead of actual data analysis, are applied to perform the design and optimization, and therefore, it is not uncommon for a completed production facility to be found sub optimal once placed into operation. Since the techniques described above for determining the kinetics of separation for interface phases provide for accurate determination of these various parameters, the use of such techniques may provide for enhanced optimization and design of production facilities.

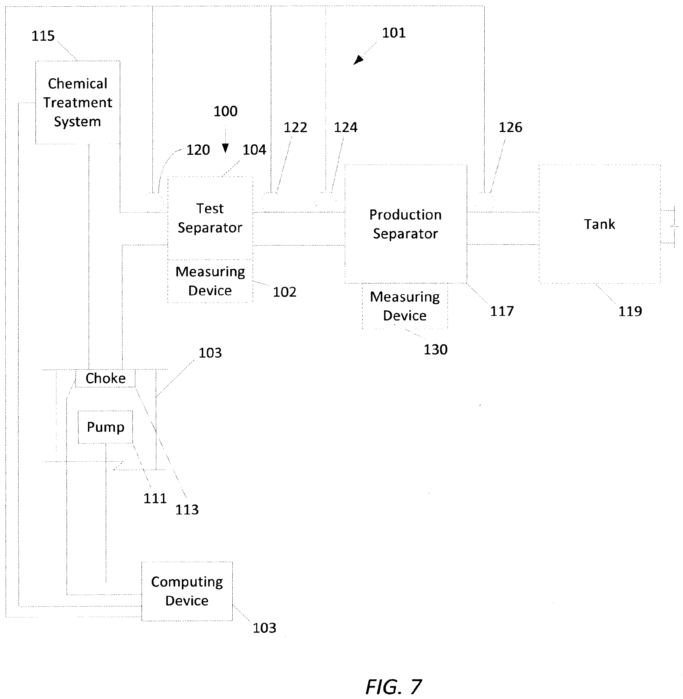

With reference to FIG. 7 a system to quantify the separability of multiphase fluids is described. A wellsite production system 101 at which the system 100 is shown. The wellsite production system 101 includes a pump 111 disposed within the wellbore 103 which serves to pump fluid out of the wellbore 103. A well choke 113 serves to help control the flow rate of the fluid from the wellbore. The pump 111 serves to pump the fluid into a production separator 117, which itself serves to separate the various phases of the multi-phasic fluid. A chemical treatment system 115 may chemically treat the fluid as it is pumped into the production separator.

As will be described below, a measuring device 102, such as a guided wave radar or other system used for collecting data about the multi-phasic fluid, phases and/or interface phases thereof, may optionally be located in an optional test separator 104. The optional test separator 104 is plumbed into the wellsite production system 101, is smaller than the production separator 117, and contains a lesser volume of fluid than the production separator 117. In the case where the optional test separator 104 is not used, a measuring device 130 similar to the measuring device 102 above may be used for collecting data about the multi-phasic fluid, phases and/or interface phases thereof, and may be located in the production separator 117.

Other possible measuring devices 102 and 130, other than guided wave radars, usable with the test separator 104 and production separator 117 may be those previously described, including, but not limited to, radar, sonar, acoustic, gravity, optical, inductance, conductance, capacitance, resistivity nuclear magnetic resonance, and nuclear measurement systems.

Optional sensors 120 and 122 may be located at the input and/or output of the optional test separator 104 and coupled to the computing device 103. Similarly, optional sensors 124 and 126 may be located at the input and/or output of the production separator and coupled to the computing device 103. It should be appreciated however that the sensors 120, 122, 124, 126 may be located anywhere useful within the wellsite production system 101.

These sensors 120, 122, 124, and 126 may be sensors used by the computing device 103 to determine the water-in-liquid ratio of the multi-phasic fluid, temperature of the multi-phasic fluid, pressure of the multi-phasic fluid, velocity of the multi-phasic fluid, flow rate of the multi-phasic fluid, or other such properties.

An optional tank 119 is coupled downstream of the production separator 117 and serves to allow for additional subsiding of the interface phase or phases in cases where the interface phase or phases have not subsided completely while in the production separator 117.

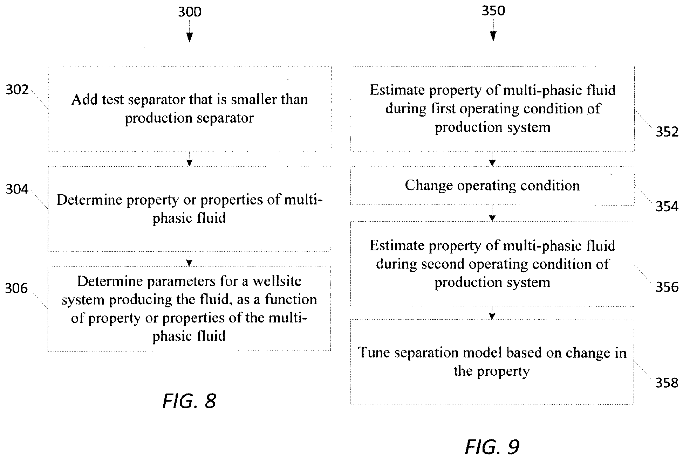

With reference to the flowchart 300 of FIG. 8, a method of applying information about the multi-phasic fluid, such as the various information about the phases collected above, is now described. As stated, a test separator 104 may optionally be added (Block 302) to the fluid flow. The test separator 104 collects fluid in parallel with the production separator 117. A property or various properties of the multi-phasic fluid is then detected and/or determined or estimated (Block 304), such as by using the measuring device 102 or measuring device 130. As an example, these properties may include the kinetics of separation of the interface phase or phases, the fraction of the multi-phasic fluid represented by each phase, the water-in-liquid ratio of the multi-phasic fluid, the density of the multi-phasic fluid, the resistivity profile of the multi-phasic fluid, the hydrogen index of the multi-phasic fluid, and/or the salinity of the multi-phasic fluid.

As explained above, determination of the various properties of the multi-phasic fluid, phases and/or interface phases thereof may be performed via any suitable method. This determination of the various properties of the multi-phasic fluid, phases and/or interface phases thereof may be performed during continuous flow of the multi-phasic fluid through the test separator 104 or production separator 117, or during a batch settling operation taking place within the test separator 104 or production separator 117. The selection of the mode (continuous or batch) may depend on the kinetics of the process and the model being used.

This determination of the various properties of the multi-phasic fluid, the phases, and/or interface phases thereof may be made on the fluid within a main compartment of the test separator 104 or production separator 117, for example, such as described above in the guided wave radar application. As an alternative, the determination of the various properties of the multi-phasic fluid, the phases, and/or interface phases thereof may be made at an outlet of the production separator 117 using the optional sensor 126, at an inlet of the production separator 117 using an optional sensor 124, at an outlet of the test separator 104, or at an inlet of the test separator 104. For instance, the sensors 120, 122, 124, or 126 may determine the temperature of the multi-phasic fluid, pressure of the multi-phasic fluid, velocity of the multi-phasic fluid, and/or flow rate of the multi-phasic fluid or a phase or interface phase thereof.

In some cases, the determination of the various properties of the multi-phasic fluid, the phases, and/or interface phases thereof may be made as a function of a composition or phase fraction of each phase of the multi-phasic fluid, which may be measured at an outlet of the production separator 117 using the optional sensor 126, at an inlet of the production separator 117 using an optional sensor 124, at an outlet of the test separator 104, or at an inlet of the test separator 104. For example, the sensors 120, 122, 124, and/or 126 may determine the water-in-liquid ratio of the multi-phasic fluid or a phase or interface phase thereof. As another example, one or more of the sensors 120, 122, 124, and/or 126 may be a multiphase flowmeter that uses nuclear technology (x-ray or gamma ray) to determine the fraction of the multi-phasic fluid represented by each phase.

Parameters for the wellsite production system 101 are then determined as a function of the property or properties of the multi-phasic fluid, such as the kinetics of separation of the interface phases (Block 306). These parameters for the wellsite production system 101 may be determined by using a model, and using the determined properties of the multi-phasic fluid as inputs to the model. From the model, or from other tools or processes, a preferred size for the production separator 117 and/or preferred type of internal mechanisms within the production separator 117 may then be determined, for example. Improper sizing of the production separator 117 may increase time used to produce usable hydrocarbons for transport downstream, or may reduce yield due to the emulsion phase not completely subsiding within the production separator 117.

Other determined parameters for the wellsite production system 101 may be a model, size, flow rate, or type of pump 111, a setting for the well choke 113, a pressure, flow rate, or temperature for the fluid, or a chemical treatment regime to be applied by the chemical treatment system 115 to the fluid. Changing the temperature for the fluid may be achieved using a heat exchanger or cooler, well choke, direct heater, or indirect heater.

Also, whether a settling tank 119 is preferred to be added downstream of and in fluid communication with the production separator 117 may be determined. For example, if it is determined that the production separator 117 is not adequately sized to permit the interface phase to subside given current operating conditions, it may be determined that the settling tank 119 is to be added. Also, the duration of time, or settling time, to be spent by the fluid in the settling tank 119 may be one of the parameters determined, as well as whether a sensor to determine a water-in-liquid ratio of fluid is to be installed in the settling tank.

The production separator 117 may be a continuous separator, where oil and water have different velocities and there is thus a continuous feed of emulsion or sludge. Continuous monitoring of the various properties of the multi-phasic fluid may be particularly useful in this case, and alarms may be triggered if the one or more properties takes on an undesirable value. For example, an alarm may be triggered if the multi-phasic fluid, or a phase or interface phase thereof, has a water-in-liquid ratio that is too high for certain purposes, such as burning. Another use for the knowledge of the various properties of the multi-phasic fluid lies in the updating of models used in designing production systems and setups, and even in the design of the models themselves. The models may be of the production systems themselves, or of properties of the production systems. In addition, the various properties of the multi-phasic fluid as determined using the techniques described above may be combined with information about the fluid collected in other fashions, such as by fluid sampling, to create a database, model, or equations which are capable of estimating the kinetics of separation and other properties of the emulsion with simple knowledge of the information collected in the other fashions.

In some applications, as will now be described with reference to FIG. 9, the various properties of the multi-phasic fluid, such as those of a phase and/or interface phase thereof (*i.e.* the emulsion phase), are estimated or determined during a first operating condition of the wellsite production system 101 (Block 352). That operating condition is then changed (Block 354) to a second operating condition, and then the various properties of the multi-phasic fluid, such as those of a phase and/or interface phase thereof (i.e. the emulsion phase), are estimated or determined again during the second operating condition (Block 356). The effect of the change from the first operating condition to the second operating condition can then be analyzed, and the separation model may be tuned based thereupon (Block 358), or process control may be changed based thereupon.

Where the production separator 117 operates in continuous separation mode, an example of an operating condition or operating conditions that may be changed are those relative to the production separator 117. In operating in accordance with a first operating condition, the velocity of the multi-phasic fluid through the production separator 117 in continuous separation mode is such that sufficient time for stabilization and separation of the multi-phasic fluid is allowed. As the multi-phasic fluid stabilizes and separates, the transient conditions of the multi-phasic fluid are monitored using the techniques described above to determine the kinetics of separation of one or more phases of the multi-phasic fluid. A steady state analysis (ignoring the transients) on the multi-phasic fluid in the production separator 117 may be used to determine separation efficiency. Conditions of operation of the production separator 117 may then be changed to a second operating condition, for example by changing operating conditions of any component or facet of the wellsite production system 101 as described above or below. Once the conditions of operation of the production separator 117 are changed to the second operating condition, the kinetics of separation and/or separation efficiency may then be determined again. Based on whether the change is considered favorable, the operating conditions of the production separator 117 may be further changed if desired.

Where the production separator 117 operates in batch settling mode, once the production separator 117 is filled to a desired level with the multi-phasic fluid, the multi-phasic fluid is then trapped in the production separator 117 by diverting flowing multi-phasic fluid around the production separator 117. This may be effectuated using suitable combinations of valves and bypass pipes, for example. Once the multi-phasic fluid is trapped, the kinetics of separation and/or separation efficiency are determined as described above. Once the batch settling is complete and the settled multi-phasic fluid has exited from the production separator, one or more operating conditions of the wellsite production system 101 may be changed as described above. A new batch of multi-phasic fluid then flows into the production separator 117, and the trapping function is repeated. Kinetics of separation and/or separation efficiency are then determined as described above. Based on whether the change is considered favorable, the operating conditions of the production separator 117 may be further changed if desired.

The operating condition that is changed may be, for example, mechanical conditions of the wellsite production system 101, such as a control scheme of the pump 111, which may change a flow rate of the pump 111, (i.e. by changing speed), and/or a fluid, pressure resulting from operation of the pump 111. Another operating condition that may be changed may be a temperature of the multi-phasic fluid, or a mechanical setting of the choke 113 (i.e. position). That is, the effect of a change in flow rate, pressure, temperature, or choke setting on the multi phasic fluid may be observed and acted upon. The change in flow rate, pressure, temperature, or choke setting may be made at any location within the wellsite production system 101, for example such as by adjusting the pressure of the multi-phasic fluid downstream of the production separator 117, using a flow restrictor or choke. As another example, the pressure of the multi-phasic fluid within the production separator 117 may be adjusted and controlled. Still further, in the case where one or more flow conditioners, such as mixers or strainers, are used within the wellsite production system 101, mechanical conditions of those flow conditioners, such as the shape, position, angle, and size of internal components of the flow conditioners may be adjusted or changed in order to change an operating condition.

The operation condition that is changed may also be a chemical treatment condition of the wellsite production system 101, such as the amounts and/or types of various chemicals injected by the chemical treatment system 115 into the fluid. Sample chemicals include de-emulsifiers, defoamers, and deviscosifiers. By monitoring the properties of the multi phasic fluid, such as those of a phase and/or interphase thereof, between different chemical treatment conditions and comparing the results of the separation efficiencies of the different chemical treatment conditions, it is possible to determine which chemicals, and in what quantities, yield the best separation of the multiphase fluids under a given set of operating conditions. Thus, it may be possible to reduce the amount of such chemicals used while obtaining similar results therefrom, resulting in a cost savings. For example, where an increase of a chemical or chemicals injected during chemical treatment of the multi-phasic fluid does not change a desired property of the multi-phasic fluid, such as kinetics of separation of interface phases, the amount of the chemical or chemicals injected may be reduced.

The operating condition that is changed may also be a volume of one or more of the phases, such that the phase ratio of the makeup of multi-phasic fluid is changed. This may be performed via the use of components of the production separator 117 under control of the computing device 103, such as valves of inlets or outlets at specific fluid levels in the production separator 117. The computing device 103 may control these valves as a function of properties of a phase or interface phase, such as the water-in-liquid ratio or thickness of the emulsion phase. As a result, the residence time of one or more of the phases within the production separator 117 may be changed and controlled. Similar to the change in volume of phases, the volume of the level of multi-phasic fluid in the production separator 117 may be an operating condition that is changed.

Yet another operating condition that may be changed may be the actual equipment of the wellsite production system 101 itself, such as the size or type of the production pump 111. As another example, part of the multi-phasic flow may be diverted from flowing into a desired component, such as the production separator 117. For example, valves may be used to send at least a portion of one or more phases of the multi-phasic fluid through a bypass pipe such that the volume and/or phase ratio of the multi-phasic fluid within the production separator 117 is changed and controlled. In addition to, or instead of, changing a volume of the phases within the production separator 117 or diverting part of the multi-phasic flow through a bypass pipe, additional fluid components such as water or other suitable fluid may be injected into the multi-phasic fluid.

By monitoring the properties of the multi phasic fluid, such as those of a phase and/or interphase thereof, between different operating conditions and comparing the results of the separation efficiencies of the different operating conditions, it is possible to determine which operating conditions yield the best separation of the multiphase fluids.

Although the above has been described with respect to the production separator 117 or test separator 104 utilizing gravity separation, in some applications electrostatic, electromagnetic, thermal, centrifugal, and distillation functionality may be used by the production separator 117 and/or the test separator 104 for separation.

The foregoing outlines features of several embodiments so that those skilled in the art may better understand aspects of the present disclosure. Those skilled in the art should appreciate that they may readily use the present disclosure as a basis for designing or modifying other processes and structures for carrying out the same purposes or achieving the same advantages of the embodiments introduced herein.

**Claims**

1.  A method (200) of determining properties of a fluid having an oil phase, a water phase, and an emulsion phase between the oil phase and water phase, the method comprising:

    emitting (202) an electromagnetic wave into the fluid using a guided wave radar (102);
    measuring (204) an amplitude of a reflection of the electromagnetic wave off an interface between the oil phase and the emulsion phase;
    determining (206) a dielectric constant of the emulsion phase based on the amplitude of the reflection of the electromagnetic wave from the interface;
    determining (208) a water-in-liquid ratio of the emulsion phase based on the dielectric constant;
    determining a height of a coalescing portion ($h_c$) of the interface between the oil phase and the emulsion phase based on the emitted and reflected waves;
    determining a height of a creaming portion ($h_s$) of the interface between the emulsion phase and the water phase based on a total height ($h_t$) of the fluid, the height of a coalescing portion ($h_c$) and the water-in-liquid ratio of the emulsion phase; and

determining (210) a thickness of the emulsion phase based on the difference between the height of the coalescing portion ($h_c$) and the height of the creaming portion *(hs)*.

2. The method of claim 1, wherein the thickness of the emulsion phase is estimated using a mass balance model.

3. The method of claim 1, wherein the dielectric constant of the emulsion phase is determined based on the amplitude of the reflection of the electromagnetic wave from the interface and a dielectric constant of the oil phase.

4. The method of claim 3, further comprising, prior to determining the dielectric constant of the emulsion phase:

measuring an amplitude of a reflection of the electromagnetic wave off an interface between free space and the oil phase; and
determining the dielectric constant of the oil phase based on the amplitude of the reflection of the electromagnetic wave off the interface between free space and the oil phase.

5. The method of claim 1, wherein the water-in-liquid ratio of the emulsion phase is determined based on the dielectric constant of the emulsion phase and a dielectric constant of at least one of the oil phase and the water phase.

6. The method of claim 1, wherein the water-in-liquid ratio of the emulsion phase is also determined based on shear rate of the emulsion phase, salinity of the emulsion phase, and continuous phase transition of the emulsion phase.

7. The method of claim 1, further comprising,

determining the total height of the fluid as a function of an elapsed time between emitting the electromagnetic wave into the fluid and receiving a reflection of the electromagnetic wave from an interface between the water phase and a body holding the fluid; and
determining the height of the coalescing portion of the interface between the oil phase and the emulsion phase as a function of an elapsed time between receiving a reflection of the electromagnetic wave from the coalescing portion and receiving a reflection of the electromagnetic wave from the interface between the water phase and the body holding the fluid.

8. The method of claim 1, further comprising determining kinetics of separation of the emulsion phase as a function of the thickness of the emulsion phase.

9. The method of claim 1, further comprising estimating a percentage of emulsion separation over time as a function of the thickness of the emulsion phase.

10. An apparatus comprising:

a vessel (104) containing a fluid having an oil phase, a water phase, and an emulsion phase between the oil phase and water phase;
a guided wave radar (102) associated with the vessel (104); and
a computing device (103) coupled to the guided wave radar (102) and configured to:

cause the guided wave radar (102) to emit an electromagnetic wave into the fluid;
measure an amplitude of a reflection of the electromagnetic wave off an interface between the oil phase and the emulsion phase, using the guided wave radar (102);
determine a dielectric constant of the emulsion phase based on the amplitude of the reflection of the electromagnetic wave from the interface;
determine a water-in-liquid ratio of the emulsion phase based on the dielectric constant; and
determine a height of a coalescing portion ($h_c$) of the interface between the oil phase and the emulsion phase based on emitted and reflected waves;
determine a height of a creaming portion ($h_s$) of the interface between the emulsion phase and the water phase based on a total height ($h_t$) of the fluid, the height of the coalescing portion ($h_c$) and the water-in-liquid ratio of the emulsion phase;
determine a thickness of the emulsion phase from the difference between the height of the coalescing portion ($h_c$) and the height of the creaming portion *(hs)*.

11. The apparatus of claim 10, wherein the vessel (104) comprises a separator (117), wherein the separator (117) has components operable by the computing device (103) and wherein the computing device (103) is configured to operate the components of the separator (117) based on the water-in-liquid ratio or the thickness of the emulsion phase.

12. The apparatus of claim 10, wherein the computing device is configured to:

determine the total height of the fluid as a function of an elapsed time between emitting the electromagnetic wave into the fluid and receiving a reflection of the electromagnetic wave from an interface between the water phase and a body holding the fluid; and
determine the height of the coalescing portion of the interface between the oil phase and the emulsion phase as a function of an elapsed time between receiving a reflection of the electromagnetic wave from the coalescing portion and receiving a reflection of the electromagnetic wave from the interface between the water phase and the body holding the fluid.

13. The apparatus of claim 10, further comprising a sensor (120, 122, 124, 126) associated with the vessel (104) and wherein the computing device is also coupled to the sensor (120, 122, 124, 126) and configured to determine the water-in-liquid ratio of the fluid based upon output from the sensor in the vessel (104).

14. The apparatus of claim 10, wherein the computing device (103) is configured to determine at least one of:

the dielectric constant of the emulsion phase based on the amplitude of the reflection of the electromagnetic wave from the interface and a dielectric constant of the oil phase;
the water-in-liquid ratio of the emulsion phase based on the dielectric constant of the emulsion phase and either a dielectric constant of the oil phase or a dielectric constant of the water phase; and
the water-in-liquid ratio of the emulsion phase based on shear rate of the emulsion phase, salinity of the emulsion phase, and continuous phase transition of the emulsion phase.

**Patentansprüche**

1. Verfahren (200) zum Bestimmen von Eigenschaften eines eine Ölphase, eine Wasserphase, und eine Emulsionsphase zwischen der Ölphase und der Wasserphase aufweisenden Fluids, wobei das Verfahren umfasst:

Aussenden (202) einer elektromagnetischen Welle in das Fluid unter Verwendung eines Guided-Wave-Radars (102);
Messen (204) einer Amplitude einer Reflexion der elektromagnetischen Welle an einer Grenzfläche zwischen der Ölphase und der Emulsionsphase;
Bestimmen (206) einer Dielektrizitätskonstante der Emulsionsphase anhand der Amplitude der Reflexion der elektromagnetischen Welle von der Grenzfläche;
Bestimmen (208) eines Wasser-in-Flüssigkeit-Verhältnisses der Emulsionsphase anhand der Dielektrizitätskonstante;
Bestimmen einer Höhe eines Koaleszenzabschnitts ($h_c$) der Grenzfläche zwischen der Ölphase und der Emulsionsphase anhand der ausgesendeten und reflektierten Wellen;
Bestimmen einer Höhe eines Aufrahmungsabschnitts ($h_s$) der Grenzfläche zwischen der Emulsionsphase und der Wasserphase anhand einer Gesamthöhe ($h_t$) des Fluids, der Höhe eines Koaleszenzabschnitts ($h_c$) und des Wasser-in-Flüssigkeit-Verhältnisses der Emulsionsphase; und
Bestimmen (210) einer Dicke der Emulsionsphase anhand der Differenz zwischen der Höhe des Koaleszenzabschnitts ($h_c$) und der Höhe des Aufrahmungsabschnitts ($h_s$).

2. Verfahren nach Anspruch 1, wobei die Dicke der Emulsionsphase unter Verwendung eines Massenbilanzmodells geschätzt wird.

3. Verfahren nach Anspruch 1, wobei die Dielektrizitätskonstante der Emulsionsphase anhand der Amplitude der Reflexion der elektromagnetischen Welle von der Grenzfläche und einer Dielektrizitätskonstante der Ölphase bestimmt wird.

4. Verfahren nach Anspruch 3, ferner umfassend, vor dem Bestimmen der Dielektrizitätskonstante der Emulsionsphase:

Messen einer Amplitude einer Reflexion der elektromagnetischen Welle an einer Grenzfläche zwischen freiem Raum und der Ölphase; und
Bestimmen der Dielektrizitätskonstante der Ölphase anhand der Amplitude der Reflexion der elektromagnetischen Welle an der Grenzfläche zwischen freiem Raum und der Ölphase.

5. Verfahren nach Anspruch 1, wobei das Wasser-in-Flüssigkeit-Verhältnis der Emulsionsphase anhand der Dielektrizitätskonstante der Emulsionsphase und einer Dielektrizitätskonstante von wenigstens einem aus der Ölphase und der Wasserphase bestimmt wird.

6. Verfahren nach Anspruch 1, wobei das Wasser-in-Flüssigkeit-Verhältnis der Emulsionsphase auch anhand von Scherrate der Emulsionsphase, Salzhaltigkeit der Emulsionsphase und kontinuierlichem Phasenübergang der Emulsionsphase bestimmt wird.

7. Verfahren nach Anspruch 1, ferner umfassend:

Bestimmen der Gesamthöhe des Fluids in Abhängigkeit von einer verstrichenen Zeit zwischen Aussenden der elektromagnetischen Welle in das Fluid und Empfangen einer Reflexion der elektromagnetischen Welle von einer Grenzfläche zwischen der Wasserphase und einem das Fluid haltenden Körper; und
Bestimmen der Höhe des Koaleszenzabschnitts der Grenzfläche zwischen der Ölphase und der Emulsionsphase in Abhängigkeit von einer verstrichenen Zeit zwischen Empfangen einer Reflexion der elektromagnetischen Welle vom Koaleszenzabschnitt und Empfangen einer Reflexion der elektromagnetischen Welle von der Grenzfläche zwischen der Wasserphase und dem das Fluid haltenden Körper.

8. Verfahren nach Anspruch 1, ferner umfassend ein Bestimmen einer Trennungskinetik der Emulsionsphase in Abhängigkeit von der Dicke der Emulsionsphase.

9. Verfahren nach Anspruch 1, ferner umfassend ein Schätzen eines Prozentsatzes an Emulsionstrennung über die Zeit in Abhängigkeit von der Dicke der Emulsionsphase.

10. Vorrichtung, umfassend:

ein Gefäß (104), das ein eine Ölphase, eine Wasserphase, und eine Emulsionsphase zwischen der Ölphase und der Wasserphase aufweisendes Fluid enthält;
ein dem Gefäß (104) zugeordnetes Guided-Wave-Radar (102); und
ein mit dem Guided-Wave-Radar (102) gekoppeltes Rechengerät (103), das dazu ausgelegt ist:

zu bewirken, dass das Guided-Wave-Radar (102) eine elektromagnetische Welle in das Fluid aussendet;
unter Verwendung des Guided-Wave-Radars (102) eine Amplitude einer Reflexion der elektromagnetischen Welle an einer Grenzfläche zwischen der Ölphase und der Emulsionsphase zu messen;
eine Dielektrizitätskonstante der Emulsionsphase anhand der Amplitude der Reflexion der elektromagnetischen Welle von der Grenzfläche zu bestimmen;
ein Wasser-in-Flüssigkeit-Verhältnis der Emulsionsphase anhand der Dielektrizitätskonstante zu bestimmen; und
eine Höhe eines Koaleszenzabschnitts ($h_c$) der Grenzfläche zwischen der Ölphase und der Emulsionsphase anhand der ausgesendeten und reflektierten Wellen zu bestimmen;
eine Höhe eines Aufrahmungsabschnitts ($h_s$) der Grenzfläche zwischen der Emulsionsphase und der Wasserphase anhand einer Gesamthöhe ($h_t$) des Fluids, der Höhe des Koaleszenzabschnitts ($h_c$) und des Wasser-in-Flüssigkeit-Verhältnisses der Emulsionsphase zu bestimmen;
eine Dicke der Emulsionsphase aus der Differenz zwischen der Höhe des Koaleszenzabschnitts ($h_c$) und der Höhe des Aufrahmungsabschnitts ($h_s$) zu bestimmen.

11. Vorrichtung nach Anspruch 10, wobei das Gefäß (104) einen Separator (117) umfasst, wobei der Separator (117) Komponenten aufweist, die vom Rechengerät (103) betreibbar sind, und wobei das Rechengerät (103) dazu ausgelegt ist, die Komponenten des Separators (117) anhand des Wasser-in-Flüssigkeit-Verhältnisses oder der Dicke der Emulsionsphase zu betreiben.

**12.** Vorrichtung nach Anspruch 10, wobei das Rechengerät dazu ausgelegt ist:

die Gesamthöhe des Fluids in Abhängigkeit von einer verstrichenen Zeit zwischen Aussenden der elektromagnetischen Welle in das Fluid und Empfangen einer Reflexion der elektromagnetischen Welle von einer Grenzfläche zwischen der Wasserphase und einem das Fluid haltenden Körper zu bestimmen; und
die Höhe des Koaleszenzabschnitts der Grenzfläche zwischen der Ölphase und der Emulsionsphase in Abhängigkeit von einer verstrichenen Zeit zwischen Empfangen einer Reflexion der elektromagnetischen Welle vom Koaleszenzabschnitt und Empfangen einer Reflexion der elektromagnetischen Welle von der Grenzfläche zwischen der Wasserphase und dem das Fluid haltenden Körper zu bestimmen.

**13.** Vorrichtung nach Anspruch 10, ferner umfassend einen dem Gefäß (104) zugeordneten Sensor (120, 122, 124, 126), und wobei das Rechengerät auch mit dem Sensor (120, 122, 124, 126) gekoppelt und dazu ausgelegt ist, das Wasser-in-Flüssigkeit-Verhältnis des Fluids anhand einer Ausgabe aus dem Sensor im Gefäß (104) zu bestimmen.

**14.** Vorrichtung nach Anspruch 10, wobei das Rechengerät (103) dazu ausgelegt ist, wenigstens eines zu bestimmen aus:

der Dielektrizitätskonstante der Emulsionsphase anhand der Amplitude der Reflexion der elektromagnetischen Welle von der Grenzfläche und einer Dielektrizitätskonstante der Ölphase;
dem Wasser-in-Flüssigkeit-Verhältnis der Emulsionsphase anhand der Dielektrizitätskonstante der Emulsionsphase und entweder einer Dielektrizitätskonstante der Ölphase oder einer Dielektrizitätskonstante der Wasserphase; und
dem Wasser-in-Flüssigkeit-Verhältnis der Emulsionsphase anhand von Scherrate der Emulsionsphase, Salzhaltigkeit der Emulsionsphase und kontinuierlichem Phasenübergang der Emulsionsphase.

**Revendications**

**1.** Procédé (200) de détermination des propriétés d'un fluide présentant une phase huileuse, une phase aqueuse et une phase d'émulsion entre la phase huileuse et la phase aqueuse, le procédé comprenant :

l'émission (202) d'une onde électromagnétique dans le fluide à l'aide d'un radar à ondes guidées (102) ;
la mesure (204) d'une amplitude d'une réflexion de l'onde électromagnétique sur une interface entre la phase huileuse et la phase d'émulsion ;
la détermination (206) d'une constante diélectrique de la phase d'émulsion sur la base de l'amplitude de la réflexion de l'onde électromagnétique à partir de l'interface ;
la détermination (208) d'un rapport eau dans liquide de la phase d'émulsion sur la base de la constante diélectrique ;
la détermination d'une hauteur d'une partie coalescente ($h_e$) de l'interface entre la phase huileuse et la phase d'émulsion sur la base des ondes émises et réfléchies ; la détermination d'une hauteur d'une partie écrémage ($h_s$) de l'interface entre la phase d'émulsion et la phase aqueuse sur la base d'une hauteur totale ($h_t$) du fluide, de la hauteur d'une partie coalescente ($h_e$) et du rapport eau-dans-liquide de la phase d'émulsion ; et
la détermination (210) d'une épaisseur de la phase d'émulsion sur la base de la différence entre la hauteur de la partie coalescente ($h_e$) et la hauteur de la partie écrémage ($h_s$).

**2.** Procédé selon la revendication 1, dans lequel l'épaisseur de la phase d'émulsion est estimée à l'aide d'un modèle de bilan massique.

**3.** Procédé selon la revendication 1, dans lequel la constante diélectrique de la phase d'émulsion est déterminée sur la base de l'amplitude de la réflexion de l'onde électromagnétique à partir de l'interface et d'une constante diélectrique de la phase huileuse.

**4.** Procédé selon la revendication 3, comprenant en outre, avant de déterminer la constante diélectrique de la phase d'émulsion :

la mesure d'une amplitude d'une réflexion de l'onde électromagnétique sur une interface entre l'espace libre et la phase huileuse ; et
la détermination de la constante diélectrique de la phase huileuse sur la base de l'amplitude de la réflexion de l'onde électromagnétique à partir de l'interface entre l'espace libre et la phase huileuse.

5. Procédé selon la revendication 1, dans lequel le rapport eau dans liquide de la phase d'émulsion est déterminé sur la base de la constante diélectrique de la phase d'émulsion et d'une constante diélectrique d'au moins une parmi la phase huileuse et la phase aqueuse.

6. Procédé selon la revendication 1, dans lequel le rapport eau dans liquide de la phase d'émulsion est également déterminé sur la base du taux de cisaillement de la phase d'émulsion, de la salinité de la phase d'émulsion et de la transition de phase continue de la phase d'émulsion.

7. Procédé selon la revendication 1 comprenant en outre,

la détermination de la hauteur totale du fluide en fonction d'un temps écoulé entre l'émission de l'onde électromagnétique dans le fluide et la réception d'une réflexion de l'onde électromagnétique à partir d'une interface entre la phase aqueuse et un corps contenant le fluide ; et
la détermination de la hauteur de la partie coalescente de l'interface entre la phase huileuse et la phase d'émulsion en fonction d'un temps écoulé entre la réception d'une réflexion de l'onde électromagnétique à partir de la partie coalescente et la réception d'une réflexion de l'onde électromagnétique à partir de l'interface entre la phase aqueuse et le corps contenant le fluide.

8. Procédé selon la revendication 1, comprenant en outre la détermination de la cinétique de séparation de la phase d'émulsion en fonction de l'épaisseur de la phase d'émulsion.

9. Procédé selon la revendication 1, comprenant en outre l'estimation d'un pourcentage de séparation d'émulsion au fil du temps en fonction de l'épaisseur de la phase d'émulsion.

10. Appareil comprenant :

un récipient (104) contenant un fluide présentant une phase huileuse, une phase aqueuse et une phase d'émulsion entre la phase huileuse et la phase aqueuse ;
un radar à ondes guidées (102) associé au récipient (104) ; et
un dispositif informatique (103) couplé au radar à ondes guidées (102) et configuré pour : amener le radar à ondes guidées (102) à émettre une onde électromagnétique dans le fluide ;
mesurer une amplitude d'une réflexion de l'onde électromagnétique à partir d'une interface entre la phase huileuse et la phase d'émulsion, à l'aide du radar à ondes guidées (102) ;
déterminer une constante diélectrique de la phase d'émulsion sur la base de l'amplitude de la réflexion de l'onde électromagnétique à partir de l'interface ;
déterminer un rapport eau dans liquide de la phase d'émulsion sur la base de la constante diélectrique ; et
déterminer une hauteur d'une partie coalescente ($h_e$) de l'interface entre la phase huileuse et la phase d'émulsion sur la base des ondes émises et réfléchies ;
déterminer une hauteur d'une partie écrémage ($h_s$) de l'interface entre la phase d'émulsion et la phase aqueuse sur la base d'une hauteur totale ($h_t$) du fluide, de la hauteur d'une partie coalescente ($h_e$) et du rapport eau dans liquide de la phase d'émulsion ; et
déterminer une épaisseur de la phase d'émulsion à partir de la différence entre la hauteur de la partie coalescente ($h_e$) et la hauteur de la partie écrémage ($h_s$).

11. Appareil selon la revendication 10, dans lequel le récipient (104) comprend un séparateur (117), dans lequel le séparateur (117) présente des éléments actionnables par le dispositif informatique (103) et dans lequel le dispositif informatique (103) est configuré pour actionner les éléments du séparateur (117) sur la base du rapport eau dans liquide ou de l'épaisseur de la phase d'émulsion.

12. Appareil selon la revendication 10, dans lequel le dispositif informatique est configuré pour :

déterminer la hauteur totale du fluide en fonction d'un temps écoulé entre l'émission de l'onde électromagnétique dans le fluide et la réception d'une réflexion de l'onde électromagnétique à partir d'une interface entre la phase aqueuse et un corps contenant le fluide ; et
déterminer la hauteur de la partie coalescente de l'interface entre la phase huileuse et la phase d'émulsion en fonction d'un temps écoulé entre la réception d'une réflexion de l'onde électromagnétique à partir de la partie coalescente et la réception d'une réflexion de l'onde électromagnétique à partir de l'interface entre la phase aqueuse et le corps contenant le fluide.

13. Appareil selon la revendication 10, comprenant en outre un capteur (120, 122, 124, 126) associé au récipient (104) et dans lequel le dispositif informatique est également couplé au capteur (120, 122, 124, 126) et configuré pour déterminer le rapport eau dans liquide du fluide sur la base de la sortie du capteur dans le récipient (104).

14. Appareil selon la revendication 10, dans lequel le dispositif informatique (103) est configuré pour déterminer au moins une parmi :

la constante diélectrique de la phase d'émulsion sur la base de l'amplitude de la réflexion de l'onde électromagnétique à partir de l'interface et d'une constante diélectrique de la phase huileuse ;
le rapport eau dans liquide de la phase d'émulsion sur la base de la constante diélectrique de la phase d'émulsion et soit d'une constante diélectrique de la phase huileuse ou une constante diélectrique de la phase aqueuse ; et
le rapport eau dans liquide de la phase d'émulsion sur la base du taux de cisaillement de la phase d'émulsion, de la salinité de la phase d'émulsion et de la transition de phase continue de la phase d'émulsion.

100

Measuring device

102

Computing
Device

103

104

Gas

Foam
Sludge

Oil

Emulsion

Water

Solid

FIG. 1

200

202 — Emit electromagnetic wave into fluid

Measure amplitude of reflection off oil surface — 205a

204 — Measure amplitude of reflection off oil-emulsion interface

Estimate dielectric constant of oil based on amplitude of reflection off oil surface — 205b

206 — Estimate dielectric constant of emulsion phase based on amplitude of reflection from oil-emulsion interface and dielectric constant of oil

Estimate total fluid height as function of elapsed time between emitting electromagnetic wave into fluid and receiving reflection from bottom of water — 209a

208 — Estimate water-in-liquid ratio of emulsion phase based on dielectric constant of emulsion and dielectric constant of oil or water

Estimate height of coalescing portion of oil-emulsion interface as function of elapsed time between receiving reflections from coalescing portion and bottom of water — 209b

210 — Estimate thickness of emulsion based on water-in-liquid ratio of emulsion phase, total fluid height, and height of coalescing portion of oil-emulsion interface

*FIG. 2*

**FIG. 3**

**FIG. 4**

*Legend:*

$h_c$ Coalescing interface
$h_s$ Creaming interface
$h_t$ Total height
$WLR_0$ Total Water Liquid Ratio

Height

$h_t$

$h_c$

$h_t WLR_0$

$h_s$

Time

**FIG. 5**

FIG. 6

*FIG. 7*

300

**302** — Add test separator that is smaller than production separator

**304** — Determine property or properties of multi-phasic fluid

**306** — Determine parameters for a wellsite system producing the fluid, as a function of property or properties of the multi-phasic fluid

*FIG. 8*

350

Estimate property of multi-phasic fluid during first operating condition of production system — **352**

Change operating condition — **354**

Estimate property of multi-phasic fluid during second operating condition of production system — **356**

Tune separation model based on change in the property — **358**

*FIG. 9*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140085133 A